Europäisches Patentamt

European Patent Office

Office européen des brevets

(11) Publication number: **0 241 725 B1**

(12) **EUROPEAN PATENT SPECIFICATION**

(45) Date of publication of patent specification: **09.12.92**

(51) Int. Cl.5: **A61K 39/39**, A61K 39/002, A61K 39/02, A61K 39/12, A61K 37/66, //(A61K37/66, 39:00)

(21) Application number: **87103717.2**

(22) Date of filing: **14.03.87**

(54) **Improvements in and relating to vaccines.**

(30) Priority: **17.03.86 GB 8606559**
**17.03.86 GB 8606560**

(43) Date of publication of application:
**21.10.87 Bulletin 87/43**

(45) Publication of the grant of the patent:
**09.12.92 Bulletin 92/50**

(84) Designated Contracting States:
**AT BE CH DE ES FR GB GR IT LI LU NL SE**

(56) References cited:
**WO-A-87/04076**

**NATURE, vol. 307, no. 5949, Jan/Feb 1984, Chesham, Bucks (GB); M.NAKAMURA et al., pp. 381-382**

**CHEMICAL ABSTRACTS, vol. 92, no. 19, 12 May 1980, Columbus, OH (US); T.A.BEKTEMIROV et al., p. 446, no. 162033q**

**LANCET, June 3, 1989, page 1264;**

(73) Proprietor: **BOEHRINGER INGELHEIM INTER-NATIONAL GmbH**
**Postfach 20**
**W-6507 Ingelheim am Rhein(DE)**

(72) Inventor: **Playfair, John Hugh Lyon, Prof.**
**5 Wetherby Gardens**
**London SW5(GB)**

Rank Xerox (UK) Business Services

BIOLOGICAL ABSTRACTS, vol. 79, no. 2, 1985, ref. 13273, P.J. GROB et al.;

BIOLOGICAL ABSTRACTS, vol. 70, no. 3, 1980, ref. 14841, I.F. BARINSKII et al.;

CHEMICAL ABSTRACTS, vol. 98, no. 19, May 9, 1983, page 367, abstractno. 158930m; Columbus, Ohio (US); I.F. BARINSKII et al.;

CHEMICAL ABSTRACTS, vol. 94, no. 11, March 16, 1981, page 547, abstract no. 81973k; Columbus, Ohio (US); H.B. LEVY et al.;

BIOLOGICAL ABSTRACTS, RRM, no. 32105964, K.J. CREMER;

CHEMICAL ABSTRACTS, vol. 106, no. 15, April 13, 1967, page 479, Columbus, Ohio (US); J.H.L. PLAYFAIR et al.;

**Description**

The present invention relates to the production of novel vaccines and to the use of those vaccines. More specifically, this invention relates to vaccines, comprising at least two components, one of which immunologically corresponds to a T cell and B cell determinant portion of an antigen, the other serves as an adjuvant.

Classically, a vaccine is manufactured by introducing a killed or attentuated organism into the host to initiate the normal immune response to the organism while, desirably, avoiding the pathogenic effects of the organism in the host.

The infectious organism/antigen against which the vaccine according to the invention is effective may be selected from any class of infectious organism, for example bacteria such as E.coli, Corynebacterium diphtheriae, Salmonella typhi, Streptococcus pneumoniae, Vibrio cholerae, Mycobacteriae, Clostridium tetani viruses such as hepatitis-, influenza-, polio-, papilloma-, foot and mouth-, rhino-, rubella-, measles-, mumps-, rabies-, varicella-, pertussis-, pox-, encephalomyocarditis- or retroviruses, protozoa such as trypanosomes or plasmodia, helminths, fungi and other organsims parasitic in the host for whom the vaccine is intended.

Viral hepatitis continues to rank as one of the most important unconquered diseases of mankind. The general term, viral hepatitis, refers principally to hepatitis B (serum hepatitis), although other known viruses and cytomegalovirus can cause hepatitis in man. Hepatitis is particularly known for its focal attack on the liver but the disease also influences other organs.

In 1965, Blomber discovered an antigen circulating in the blood of certain human beings [J Am. Med. Assoc., 191, 541 (1965) and Ann. Int. Med., 66, 924 (1967)]. This substance was subsequently found by Prince to be surface antigen of hepatitis B virus (HBsAg) that is produced in abundance by individuals who are chronically infected with the agent [Proc. Natl. Acad. Sci. (USA), 60, 814 (1968)].

There is an urgent need for a hepatitis B vaccine for groups which are at an increased risk of acquiring this infection. These groups include health care and laboratory personnel, and individuals requiring (1) maintenance hemodialyse; (2) repeated blood transformations or the administration of blood products; (3) treatment with immunosuppressive or cytotoxic drugs and (4) treatment for malignant diseases and disorders associated with depression of the immune response. In addition, a vaccine is needed for individuals living in certain tropical areas where hepatitis B infection is prevalent.

Current vaccines for HBV consist of subviral components of the virus surface coat (HBsAg) purified from the plasma of chronically HBV-infected donors and inactivated [McAuliffe et al.,Rev. Infect. Dis. 2, 470 (1980)]. Clinical trials have demonstrated the safety and efficacy of current HBsAg vaccines but such vaccines are limited in supply and are relatively expensive, particularly for those countries with the highest incidence of HBV disease.

The invention concerns improvements in and relating to vaccines.

Malaria is a severe, widespread disease for which, despite years of extensive efforts, a vaccine has not been developed. See, for example, Science, Volume 226, page 679 (November 9, 1984). Experimentally, mammals, including man, have been protected against infection by the etiologic agent of malaria, Plasmodium, by vaccination with irradiated sporozoites. Clyde et al., Am. J. Trop. Med. Hyg., 24, 397 (1975) and Rieckman et al., Bull, WHO, 57 (Supp. 1), 261 (1979). Yoshida et al., Science, 207, 71 (1980) report that such protection is at least partially mediated by antibody directed against a protein on the surface of the sporozoite, the circumsporozoite (CS) protein; monoclonal antibodies raised against CS proteins neutralize infectivity in vitro and protect animals in vivo. The CS protein appears to be highly evolutionarily conserved within species, but is quite varied across species.

Various species of Plasmodium are known to infect man. These are P. falciparum, P. vivax P. yoelii, P. ovale P. malariae, the latter two occurring at much lower frequency. Other species of scientific interest are P. berghei and P. knowlesi, the host of these species being, respectively, rodents and monkeys.

Hopes are currently high that some form of human malaria vaccine will shortly be under test, but it will probably be a considerable time before a particular antigen, or even a single stage, emerges as the first choice (McGregor I. Parasitology Today; 1, 1-33 (1985)). Rhinoviruses are RNA viruses and, according to the conventional classification of viruses, they form a genus within the family of the Picorna viruses.

They are widespread, attack the upper respiratory tract in humans and cause acute infections which lead to colds, coughs, hoaeseness etc. and are generally referred to as colds. Infections caused by rhinoviruses are among the most common sicknesses in man. Although the course of these diseases is generally harmless, colds do result in a temporary weakening of the organism. This may give rise to secondary infections caused by other viruses or bacteria which may, under certain circumstances, result in serious illness. In addition, the economic damage caused by rhinoviruses is considerable. It has been calculated that in the USA rhinovirus infections annually cause the loss of more than 200 million working days or school days. In addition, in recent years, there has been a considerable increase in rhinovirus infections in large conurbations. Whereas most other infectious diseases confer a permanent or longlasting immunity from the pathogen in question, infections caused by rhinoviruses may recur again and again. The reason for the absence of any lasting immunity is the large number of strains of rhinovirus. Hitherto, over 100 rhinovirus strains have been isolated, showing no or very few immunological cross-reactions with each other. After the infection has occurred, antibodies against the particular virus strain can be detected, but these confer no protection against other rhinovirus strains. Because of the large number of strains circulating in the population, repeated infections by rhinoviruses are possible.

One object of the EPA 0 192 175 was therefore to prepare agents which give protection from infections caused by rhinoviruses.

These may then be used to stimulate an immune response directed against intact viruses. Studies of the use of oligopeptides for stimulating an immune response directed against polio viruses have been published (Emini, E.A., Jameson, B.A. and Wimmer, E., Nature (London) 30, 699-703, (1983); similar investigations have also been carried out in the case of foot and mouth viruses (Bittle, J.L., Houghton, R.A., Alexander, H., Shinnick, T.M., Sutcliffe, J.G., Lerner, R.A., Rowlands, D.J. and Brown, F., Nature (London), 298, 30 - 33, (1982); Pfaff, E., Mussgay, M. Böhm, H.O., Schulz, G.E. and Schaller, H., EMBO J. 1, 869 - 874, (1982)).

Many gastro-enteric diseases in humans and animals, such as those caused by Escherichia coli and similar bacteria, are generally the result of toxin production which operates to disrupt the fluid balance in the gastro-intestinal tract. The result is excessive production of fluids and elektrolytes from the epithelial cells in the gastro-intestinal tract. (Moon, J.W., Adv. Vet. Sci. and Compt. Med., 18:179-211 (1974)). By way of example, certain strains of E.coli cause cholera like diseases in humans and young farm animals, which may result from the action of either of two toxins that have been isolated and identified as ST, which is heat stable, and LT, which is heat labile. (Kohler, E.M., Am. J. Vet. Res.. 29:2263-2274, (1968): Gyles, C.L. & Barnum, D.R., J. Inf. Dis. 120:419-426 (1968)).

Recent studies have shown that in order for pathogens to successfully colonize parts of the body, it is necessary for the pathogen to have the ability to adhere to the cell surfaces so that it can multiply. After colonization, the pathogens produce agents, such as toxins, which cause the undesirable symptoms. (Science 209:1103-1106, Sept. 1980). The adhesins necessary for this adherence are typically structures called pili. which are thread-like projections on bacterial cell surfaces and are typically necessary if the pathogen is to cause disease.

It has been established that immunity to pathogens can be conferred by immunizing against the toxins which they produce; Dobrescu, L., and Huygelen, C., Zpl. Det. Med. B, 23:79-88 (1976), and by immunizing against the adhesin factor, Jones, G.W., and Rutter, J.M., Am. J. of Clinical Nutrition, 27:1441-1449, (Dec. 1974); Nagy, B., Infect. Immun., 27:21-24, (Jan. 1980). Vaccines conferring such immunities have been prepared with killed pathogens which themselves contained genes for producing the offending substances. In addition, vaccines have been prepared using the pure adhesin itself.

Many efforts are in progress to prepare vaccines for Acquired Immuno-deficiency Syndrome. Recently Capon et al. described molecularly cloned acquired immunodeficiency syndrome polypeptides and a vaccine against this syndrome which confers resistance to infection by acquired immunodeficiency syndrome associated retroviruses (EPA 01 87 041).

It is well known in the vaccine art that the administration of such antigens frequently results in relatively little immunitiy unless a further component, known as an adjuvant, is also present in the vaccine composition.

The vaccines with which the invention is concerned are primarily those which comprise an antigen effective to promote specific immunity against an infective organism, or against any biologically active substance whose activity is to be controlled (e.g. a hormone).

Many vaccines are complex and include not only the antigenic determinant of interest but many related and unrelated deleterious materials, any number of which may, in some or all individuals, induce an undesirable reaction in the host.

In the past, antigens have been obtained by several methods including derivation from natural materials, coupling of a hapten to a carrier and by recombinant DNA technology. Sela et al. [Proc. Nat. Acad. Sci. (USA), 68, 1450 (1971); Science, 166, 1365 (1969); and Adv. Immun., 5, 129 (1966)] have also described certain synthetic antigens.

Certain "synthetic" antigens have been prepared by coupling small molecules (for example, dinitrophenol) to carriers (such as bovine serum albumin), thus producing antigens which cause the production of antibody to the coupled small molecule. The carrier molecule is often necessary because the small molecule itself may not be "recognized" by the immune system of the animal into which it is injected. This techniques has also been employed in isolated instances to prepare antigens by coupling polypeptide fragments of known proteins to carriers, as described in the above-referenced Sela et al.articles.

Chemically synthesized polypeptides offer considerable advantages in terms of cost and safety of vaccination programs.

It is known that antisera to synthetic polypeptid from the nucleotide sequence of various regions of the S gene of HBV react with native HBsAg by radioimmunoprecipitation [Lerner et al., Proc. Natl. Acad. Sci. (USA), 78, 3403 (1981)] and commercial solid-phase radioimmunoassays for anti-HBsAg [Gerin et al., Viral Hepatitis, Szumess et al (eds.), 49-55 (1982)].

Before synthetic vaccines become an alternative to conventional ones ways must be found to increase the magnitude and duration of the immunity they confer. An advantage of attenuated viruses is that they keep proliferating and thus present ever more antigen to the immune system, thereby increasing the antibody level. A synthetic peptide, like a killed virus, does not replicate, and so substances called adjuvants must be added to the vaccine to increase the level and duration of immunity.

Some highly effective adjuvants have been identified in animal test, such as Freund's Complete Adjuvant, the dipeptide known as MDP, saponin, aluminium hydroxide, and Bordetella pertussis. However, such materials are not of proven efficacy, such as Freund's Complete Adjuvant and saponin, are liably to cause more or less severe ulceration at the site of injection, or other serious side effects, and are not acceptable for human pharmacology.

One of the most powerful of these Freund's adjuvant, is an emulsion of mineral oil and water mixed with killed bacteria (mycobacteria, a class that includes the agent of tuberculosis). Just how it works is not known. Powerful adjuvants such as Freund's adjuvant may not be administered to the human beings, instead, alum, or aluminium hydroxide, is commonly included with a killed-virus vaccine. The antigen is adsorbed onto the aluminium hydroxide particles and is released slowly after injection. In some cases, it appears alum will serve well enough with synthetic vaccines, but most such vaccines will require something better. Much efforts is currently aimed at the development of safe and effective adjuvants. Some hopeful results in this direction have been reported by Francois Audibert and Louis Chedid of the Pasteur Institute and Ruth Arnon and Michael Sela of the Weizmann Institute of Science, who recently elicited antibodies conferring passive immunity against th diphteria toxin. They synthesized three peptides corresponding to regions of the toxin molecule. Before injection a synthetic peptide must be coupled to a carrier molecule. In some experiments the carrier was keyhole-limpet hemocyanin, a respiratory pigment of a mollusk, which is often used for this purpose. The Pasteur-Weizmann group devised a carrier-adjuvant combination. They coupled their diphtheriatoxin peptides to a carrier that had been linked to a simple derivative of the cell membrane derivative of the cell membrane of mycobacteria; the membrane derivative apparently serves as an adjuvant, significantly increasing the immunogenicity of the peptides. From the above it is clear that there is a need for a vaccine adjuvant capable of potentiating the immune response induced by an antigen without the untoward side-effects of many known adjuvants.

We have now found that interferon γ is an effective component which enhances immune response and thereby acts like an adjuvant. Thus in one aspect our invention provides IFN γ for use as a vaccine adjuvant. Another aspect of our invention provides a vaccine comprising an antigen effective to promote specific immunity against an infectious organism or against any biologically active substance whose activity is to be controlled (e.g. a hormone) and as adjuvant an effective amount of interferon.

In one aspect the invention is a vaccine against infection by an infectious organism, comprising an effective amount of an antigenic and immunogenic preparation of an infectious organism for example bacteria, viruses, protozoa, for whom the vaccine is intended, interferon gamma as adjuvant and a physiologically tolerable carrier and/or diluent if necessary, said vaccine when introduced into a host, being capable of inducing the production of antibodies and stimulating B- and/or T-cells in the host, said antibodies immunoreacting with said infectious organisms and said vaccine protecting the host from infection.

In one aspect the invention is a vaccine against infection by viruses such as hepatitis-, influenza-, polio-, papilloma-, foot and mouth-, rhino-, rubella-, measles-, mumps-, rabies-, varicella-, pertussis-, pox-, encephalomyocarditis- or retroviruses comprising an effective amount of a polypeptide that immunologically corresponds substantially to portions of the virus, as adjuvant interferon gamma and a physiologically tolerable carrier and/or diluent if necessary, said vaccine when introduced into a host, being capable of inducing the production of antibodies and stimulating B- and/or T-cells in the host, said antibodies immunoreacting with said virus and said vaccine protecting the host from said viral infection.

In one aspect the invention is a vaccine against infection by protozoa such as trypanosomes or plasmodia comprising an effective amount of an antigenic and immunogenic preparation of the infectious organism, as adjuvant interferon gamma and a physiologically tolerable carrier and/or diluent if necessary said vaccine when introduced into a host, being capable of inducing the production of antibodies and stimulating B- and/or T-cells in the host, said antibodies immunoreacting with said protozoon and said vaccine protecting the host from protozoa infection.

In one aspect the invention is a vaccine against infection by bacteria such as E.coli, Corynebacterium diphtheriae, Salmonella typhi, Streptococcus pneumoniae, Vibrio cholerae, Mycobacteriae, Clostridium tetani comprising an effective amount of an antigenic and immunogenic preparation of the infectious organism, as adjuvant an interferon gamma and a physiologically tolerable carrier and/or diluent if necessary said vaccine when introduced into a host, being capable of inducing the production of antibodies and stimulating B- and/or T-cells in the host, said antibodies immunoreacting with said bacterium and said vaccine protecting the host from bacterial infection.

The invention further comprises methods of preparing vaccines according to the invention and kits for said vaccines.

For use in a vaccine intented for administration to humans, the interferon is most preferably human interferon produced by recombinant DNA techniques; such material is now commercially available, for example from Boehringer Ingelheim. For veterinary use, the interferon $\gamma$ is preferably more or less closely related to the interferon found naturally in the species for whom the vaccine is intended, although again it will generally be of recombinant origin.

The infectious organism or antigen can be further purified such as by addition of a selective precipitating agent, followed by a final chromatographic step such as ion exchange chromatography or reverse phase HPLC.

In the vaccine of the invention, an aqueous solution of the infectious organism preferably buffered at physiological pH, can be used directly. Alternatively, the polypeptide can be encapsulated within microparticles such as liposomes. It is also possible to use combinations of various infectious organism in one vaccine.

The amount of antigen present in each vaccine dose is selected as an amount which induces an immunoprotective response without significant, adverse side effects in typical vaccinees. Such amount will vary depending upon which specific antigen is employed and whether or not the vaccine is further adjuvanted. Generally, it is expected that each dose will comprise 1 - 1000 $\mu$g of antigen, preferably 10 - 200 $\mu$g. An optimal amount for a particular vaccine can be ascertained by standard studies involving observation of antibody titres and other responses in subjects.

We have noted a particularly strong adjuvant effect with gamma interferon. The amount of interferon required to produce the desired adjuvant effect should be found by experiment, but typically may lie in the range 100 - 50,000 units, e.g. 1.000 - 10,000 units per dose of vaccine. It is preferred to include the interferon in the vaccine composition. Alternatively the interferon and antigen may be separately administered, preferably to the same site, in such manner that the desired adjuvant effect is obtained. For this purpose the antigen and adjuvant may be formulated in a two-part pack, e.g. in separate ampoules or the like. It is believed that the adjuvant effect of the interferon is particularly valuable in the case of the first dose of vaccine to be administered.

Vaccine preparation is generally described in New Trends and Developments in Vaccines, edited by Voller et al., University Park Press, Baltimore, Maryland. U.S.A., 1978. Encapsulation within liposomes is described, for example, by Fullerton. U.S. Patent 4,235,877.

Vaccines according to the invention may be formulated in conventional manner, e.g. with sterile physiologically compatible carriers such as saline solution, excipients, other adjuvants, preservatives, stabilisers, and the like. Injectable preparations will normally be formulated to be substantially isotonic. Vaccines may be in liquid form, or may be in dry form for dissolution in a sterile carrier prior to use. Vaccines may be single-dose preparations, or may be in other forms such as multi-dose flasks for use in mass vaccination programmes. Preparations effective by subcutaneous administration will often be preferred on account of their ease of use, although other administration routes such as intravenous, intramuscular and intradermal are also contemplated.

If necessary, following an initial vaccination, subjects will preferably receive a boost in about 4 weeks, followed by repeated boosts every six months for as long as a risk of infection exists. The infectious organism against which the vaccine is effective may be selected from any class of infectious organism. An infectious organism of particular interest and importance is Plasmodium spp, e.g. the human malaria parasite P. falciparum and the lethal rodent parasite P. yoelii. In tests with vaccines for the latter organism in mice we have shown, as is demonstrated in the tests below, that IFN-gamma not only produces a powerful adjuvant effect, superior even to that given by B. pertussis, but promotes a more rapid immunological reaction to infection such that an aparasitanaemic condition is achieved far more rapidly than by use of the known adjuvant.

Since there is at present no convenient way to purify antigens by virtue of their T-cell stimulating properties, we have adopted the alternative strategy of first obtaining a highly protective soluble antigen preparation, and then testing the protective effect of individual components, without reference to their reactivity with antibodies. Once an antigen is isolated, its reactivity with both T and B cells can be evaluated and correlations with protectivity looked for. In the present invention we describe a simple and reliable soluble vaccine giving effective protection against the highly virulent YM strain P. yoelii. Given intraperitoneally with saponin it is somewhat less effective than a formalin-fixed parasite vaccine given intravenously with B.pertussis, which requires only one rather than two injections (Playfair et al., Immunology, 33, 507-515 (1977)). However the soluble vaccine is also quite effective when given subcutaneously or intramuscularly, which is of obvious relevance to human vaccination. Moreover, a comparison of the dose-response relationship given in Table 1 with that found for the 230,000 MW purified P.yoelii protein (Freeman & Holder, Clin. exp. Immunol., 54, 609-616 (1983)) both of which begin to lose full protectivity in the 1-2.5 µg range, suggests that the 230,000 MW protein is not the only protective antigen in our lysate.

An encouraging feature of these experiments has been the potency of recombinant IFN-$\gamma$ as an adjuvant for subcutaneous use; this appears to be the first report of the ability of IFN to act as an adjuvant by enhancing priming. In this aspect IFN seems to stimulate immunological memory, i.e. using the conceptional framework of the clonal selection theory (Burnet. F.M., The Clonal Selection Theory of Acquired Immunity. Nashville Tn.; Vanderbilt University Press, 1959) this effect could be explained as if virgin T- and B-cells are stimulated by IFN to multiply and differentiate into memory cells. Therefore the term adjuvant according to the invention only stands for describing an effect which could be explained as if immunological memory is enhanced but neither this explanation nor the term adjuvant should in any way be used to limit the present invention.

For convenience in describing the degree of protection in 250 mice we have scored protection by vaccines as "early" or "late", since, in this experimental model, challenged mice either recover on days 8 - 10 (early) or between 15 - 20 (late). Table 1 shows all the protection data. Vaccinated mice given antigen (lysate) alone showed only occasional late protection, the subcutaneous route being somewhat better than intraperitoneal. As described earlier (Playfair & De Souza, Parasite Immunol., 8 409, (1986)) antigen i.p. plus saponin protected all the mice almost invariably by day 10.

The adjuvant effect of $\gamma$-IFN was only slightly inferior to that of saponin. With 5000 u, the dose used in most experiments, over 90% of the mice were protected including over 70% which recovered early: the intraperitoneal and subcutaneous routes were equally effective. Intradermal and intramuscular injection were also effectiv but somewhat less so. In a smaller series of experiments doses of IFN down to 200 units were also effective.

An important consideration with all recombinant materials is whether the effects are due to contaminating endotoxin, particularly since lipopolysaccharide (LPS) is known to have adjuvant avtivity. We do not believe that the effects reported here are due to LPS since the endotoxin content (by Limulus assay) of the IFN was less than 0.25 EU/mg protein, which is equivalent to less than 0.1 pg per 5000 units of $\gamma$-IFN. As Table 1 shows, doses of LPS well above this range had only weak adjuvant activity in our model and never induced strong early protection. Saponin and $\gamma$-IFN alone were not protective, so it is unlikely that the $\gamma$-IFN is acting directly in the blood-stage parasites as has recently been shown for the liver stage (Ferreira et al., Science, 232, 881 (1986)).

While the above fully describes the invention and all preferred embodiments thereof, it is to be appreciated that the invention is not limited to the embodiments particularly described but rather includes all modifications thereof coming within the scope of the following claims.

Throughout this application, the terms "peptide" and "polypeptide" are used interchangeably. As used herein, the term "synthetic polypeptide" means a chemically built-up.

Throughout the application, the phrase "immunologically corresponds substantially" is used herein in relation to the antigens to mean that the antigen induces production of antibodies that bind to the antigen and (a) bind to the antigenic determinant of the native infectious organism or (b) induce T cell proliferation. Thus, the antigens of this invention function immunologically as do the corresponding infectious organism while also being capable of inducing the production of antibodies to themselves.

The word "antigen" has been used historically to designate an entity that is bound by an antibody and to designate the entity that induces the production of the antibody. More current usage limits the meaning of antigen to that entity bound by an antibody, while the word "immunogen" is used for the entity that induces antibody production. In some instances, the antigen and immunogen are the same entity. Where an entity discussed herein is both immunogenic and antigenic, it will generally be termed an antigen, infectious organism or biologically active substance.

Legends to the Figures:

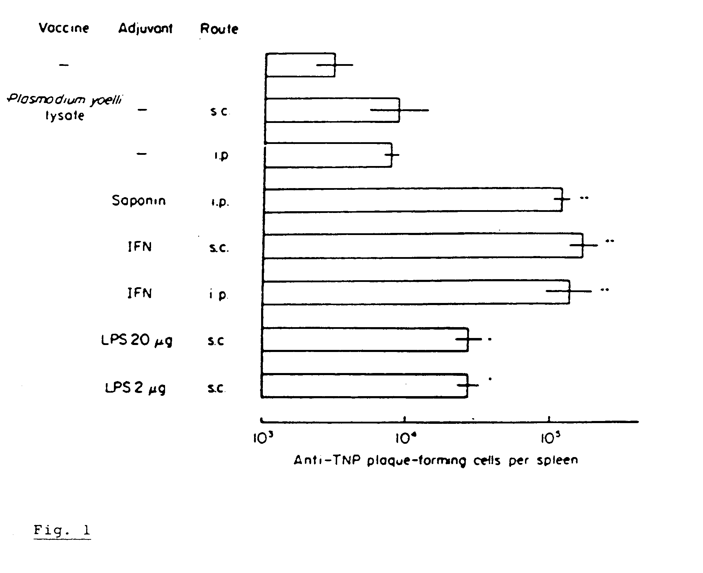

Figure 1 shows the total IFA titres 10 days after challenge. Mice vaccinated with antigen alone had titres of 1/256-1/1000 whilst both saponin and γ-IFN boosted these 8-16-fold. Intraperitoneal injection appeared marginally better.

Figure 2 shows that mice vaccinated with antigen alone gave DTH responses no stronger than unvaccinated mice, but that both saponin and γ-IFN, given subcutaneously, induced strong priming for DTH. Saponin i.p. (the most protective combination) induced the strongest DTH but, surprisingly γ-IFN by this route did not induce significant DTH.

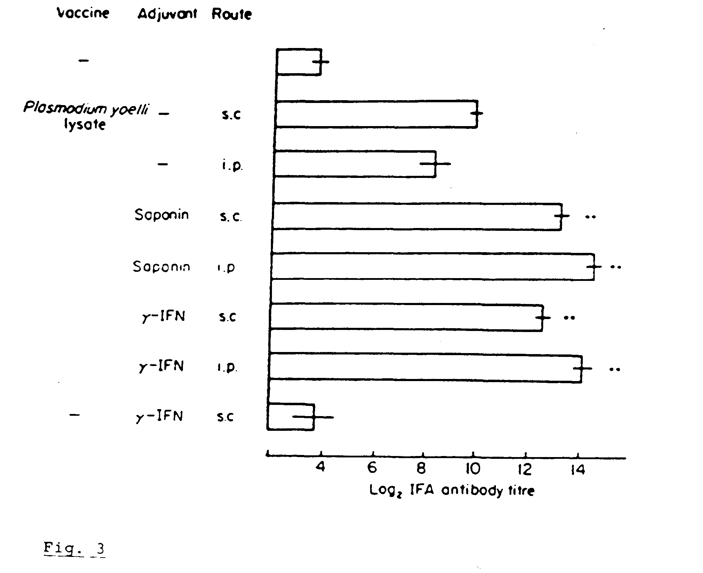

Figure 3 shows that both saponin and γ-IFN induced excellent T helper priming. In this case γ-IFN was equally effective by the subcutaneous and intraperitoneal routes.

The following embodiment of our invention is given by way of example and illustration.

Mice

(C57 Bl x Balb/c) Fl mice of both sexes were used at 10-14 weeks of age.

Parasites

The virulent YM line of P.yoelii (A.A. Holder and R.R. Freeman. Nature 294, 361-364) was maintained by weekly blood passage. Mice were infected by i.v. injection of $10^4$ parasitised red cells. Parasitaemias were counted on Giemsa-stained tail blood films.

Preparation of vaccines

Donor mice infected i.v.with $10^7$ parasitised red blood cells were bled 4 days later into heparinized phosphate buffered saline (PBS), when their parasitaemias were approaching 100%, 90% of the parasites being schizonts. After three washes in PBS the parasitised blood was lysed on 0.01% saponin at 37°C for 30 min and washed three times or until the supernatant was free of visible haemoglobin. The pellet was resuspended in extraction buffer (see below) at a ratio of 1 vol pellet to 5 vols buffer. Solubilization was carried out at 4°C for 3 hrs with vortexing at intervals. Cell debris and insoluble material were removed by microfuging for 10 mins. The supernatant was then dialysed overnight against PBS before being used to immunise mice as described below.

Extraction buffer

Triton® X-100 was used at 0.5%, made up in 50 mM tris-HCl at pH 8.0, with 5 mM EDTA, 20 mM iodoacetamide, 5 mM PMSF, 1 μg/ml pepstatin (all from Sigma) and 5 μl/ml Trasylol, as described by Deans et al (Clin. Exp. Immunol. 49, 297-309 (1982)), with the addition of 2mcg/ml of leupeptin (Calbiochem).

Vaccination and adjuvant

Mice were vaccinated with two intraperitoneal injections of 25 μg of lysate protein at 2-week intervals, either with no adjuvant (control), or with Bordetella pertussis ($10^8$ organism) or murine IFN-gamma (batch 3209-14 and 3209-33, specific activity $1.5 \times 10^7$ U/mg, purity > 99%, endotoxin content < 0.25 EU/mg) supplied by Boehringer Ingelheim. We also investigated other routes of injection, namely subcutaneous, intramuscular and intradermal.

The mice were challenged three weeks later with $10^4$ parasites i.v. The results are shown in Table II.

Patterns of recovery in vaccinated mice

All unvaccinated mice were dead by day 9. Vaccinated mice fell into two clear groups: those which recovered by day 7-10 and those which recovered around day 17-20. The latter group always showed a fall in parasitaemia on day 6 but failed to maintain this. For the purposes of the present study, we have therefore classified protection by vaccines as "early" if parasitaemia was permanently cleared on or before day 10, and "late" if it occurred subsequently. In fact "late" recovery in these experiments almost invariably occurred between days 17 and 25.

It will be seen that IFN-gamma as adjuvant gave excellent protection, especially by the desirable s.c. route. Moreover 15 out of 19 mice in this group recovered early, whereas in the corresponding test with B. pertussis adjuvant 4 mice recovered early, 4 recovered late and 2 died. Without adjuvant, 17 out of 20 mice died. Further trials (results not shown) indicated that the adjuvant alone gave no protection.

Assay for T help

Mice were vaccinated as described above, and 3 weeks after the last dose of vaccine they were injected intravenously with $10^5$ red blood cells parasitized with P.yoelii, coated with the hapten trinitophenol (TNP) by incubation with 2,4,6-trinitrobenzene sulphonic acid (Playfair, De Souza & Cottrell, Immunology, 32, 681 (1977)) (TNP-PY), or with TNP-coated normal mouse red blood cells (TNP-MRBC). Four days later their spleens were assayed for direct anti-TNP plaque-forming cells (PFC) in Cunningham chambers (Playfair et al., Immunology 32, 681 (1977)). Because we had previously shown that T helper cell priming is maximal with small doses of vaccine, we routinely used a single injection of 1 $\mu$g of antigen.

Assay for delayed hypersensitivity

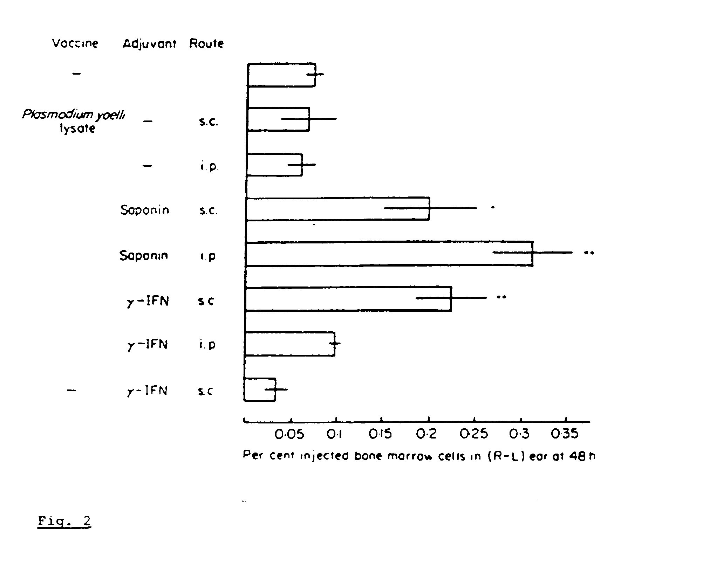

Mice were vaccinated as described above, and 3 weeks after the last dose of vaccine they were injected in the pinna of the right car with $3 \times 10^6$ red blood cells parasitized with P.yoelii in 10 $\mu$l of phosphate buffered saline (PBS) and in the left (control) ear with PBS only. One day later they were injected intraveneously with $10^7$ $^{51}$Cr-labelled normal syngeneic bone marrow cells and after another 24 h they were killed, their ears were cut off and their radioactivity was counted on an LKB Gamma counter (Cottrell. Playfair & De Souza, Clin. exp. Immunol., 34, 147 (1978)). The results shown in Fig. 2 are the percentage of the total injected bone marrow cells homing specifically to the antigen-challenged ear, calculated as:

$$\frac{(ct/min\ in\ right\ ear) - (ct/min\ in\ left\ ear)}{Total\ ct/min\ in\ bone\ marrow\ injection} \times 100$$

Antibody

Antibody responses following challenge were measured by indirect fluorescence (IFA) using the slide method of Voller & O'Neill Bulletin of the World Health Organization, 45, 524 (1971). In vaccinated mice, this assay detects predominantly IgG antibody (Playfair & De Souza, Parasite Immunol. 1 197 (1979)).

Table 1

| Effect of adjuvants on protection | | | | | | | |
|---|---|---|---|---|---|---|---|
| Vaccine | Adjuvant | Dose | Route | Recovered | | Died | Per cent protected |
| | | | | Early | Late | | |
| - *P. yoelii* lysate | - | 25 μg | s.c. | 0 0 | 0 4 | 32 28 | 0 12•5 |
| | - | | i.p. | 0 | 0 | 19 | 0 |
| | - | | i.m. | 0 | 2 | 12 | 14•3 |
| | Saponin | 25 μg | s.c. | 7 | 2 | 2 | 82 |
| | | | i.p. | 36 | 2 | 0 | 100 |
| | γ-IFN | 5000 u | s.c. | 25 | 6 | 3 | 91 |
| | | | i.p. | 14 | 2 | 2 | 89 |
| | | 2000 u | s.c. | 2 | 2 | 1 | 80 |
| | | | s.c. | 2 | 2 | 0 | 100 |
| | | 1000 u | s.c | 5 | 4 | 3 | 75 |
| | | | i.p | 2 | 3 | 0 | 100 |
| | | 500 u | s.c. | 3 | 1 | 1 | 80 |
| | | | i.p. | 4 | 0 | 1 | 80 |
| | | 200 u | s.c. | 2 | 1 | 2 | 60 |
| | | | i.p. | 1 | 3 | 1 | 80 |
| | | 100 u | s.c. | 1 | 0 | 4 | 20 |
| | | | i.p. | 3 | 0 | 2 | 60 |
| | LPS | 20 μg | s.c. | 0 | 3 | 4 | 43 |
| | | 2 μg | s.c. | 0 | 3 | 3 | 50 |
| | | 200 ng | s.c | 1 | 2 | 7 | 43 |
| | | 20 ng | s.c. | 1 | 1 | 4 | 33 |
| | | 2 ng | s.c. | 0 | 0 | 3 | 0 |
| | | 200 pg | s.c. | 0 | 0 | 2 | 0 |
| - | Saponin | 25 μg | i.p. | 0 | 0 | 6 | 0 |
| - | γ-IFN | 5000 u | s.c. | 0 | 0 | 6 | 0 |
| - | | | i.p. | 0 | 0 | 6 | 0 |
| Mice were given two injections 2 weeks apart of vaccine and adjuvant by the route indicated. The results are the numbers of mice which cleared their parasitaemia early (< 10 days) or late (> 10 days) or died.following a challenge of $10^4$ parasitized red cells. The total per cent of mice protected is also shown. | | | | | | | |

Table II

| Effect of route and adjuvant on protection by a Triton® X-100 P.yoelli lyasate | | | | | |
|---|---|---|---|---|---|
| Adjuvant | Route | Recovered | | Died | % protected |
| | | Early | Late | | |
| IFN-gamma 5000 units | i.p. | 4 | 2 | 0 | 90 |
| | s.c. | 15 | 4 | 0 | |
| | i.m. | 2 | 4 | 2 | |
| | i.d. | 2 | 3 | 2 | |
| B.pertussis $10^8$ organisms | s.c. | 4 | 4 | 2 | 75 |
| | i.m. | 4 | 3 | 1 | |
| | i.d. | 2 | 4 | 4 | |
| nil | i.p. | 0 | 2 | 2 | 17 |
| | s.c. | 0 | 3 | 17 | |
| | i.m. | 0 | 2 | 9 | |
| | i.d. | 0 | 0 | 6 | |

**Claims**

1. A vaccine for effective immunization of a mammal against infection by an infectious organism parasitic in the host, said organism being a protozoon, a virus or a bacterium, comprising an effective amount of an antigenic and immunogenic preparation of the infectious organism or of the biologically active substance, interferon-gamma (IFN$\gamma$) as an adjuvant and a physiologically tolerable carrier and/or diluent, said vaccine when introduced into the host, being capable of inducing the production of antibodies and priming memory cells in the host, said antibodies immunoreacting with said infectious organism or with the biologically active substance and said vaccine protecting the host from infection.

2. A vaccine as claimed in claim 1 against infection by hepatitis B virus comprising an effective amount of a polypeptide that immunologically corresponds to portions of the hepatitis B virus surface antigen, interferon-gamma as an adjuvant and a physiologically tolerable carrier and/or diluent, said vaccine when introduced into a host, being capable of inducing the production of antibodies and priming memory cells in the host, said antibodies immunoreacting with said hepatitis B virus and said vaccine protecting the host from hepatitis B viral infection.

3. A vaccine as claimed in claim 1 against infection by tetanus-bacterium comprising an effective amount of an antigenic and immunogenic preparation of the tetanus-bacterium, interferon-gamma as an adjuvant and a physiologically tolerable carrier and/or diluent, said vaccine when introduced into a host, being capable of inducing the production of antibodies and priming memory cells in the host, said antibodies immunoreacting with said tetanus-bacterium and said vaccine protecting the host from tetanus-bacterial infection.

4. A vaccine as claimed in claim 1 against infection by influenza virus comprising an effective amount of a polypeptide that immunologically corresponds to portions of the influenza virus, interferon-gamma as an adjuvant and a physiologically tolerable carrier and/or diluent, said vaccine when introduced into a host, being capable of inducing the production of antibodies and priming memory cells in the host, said antibodies immunoreacting with said influenza-virus and said vaccine protecting the host from influenza viral infection.

5. A vaccine as claimed in claim 1 against infection by rhinovirus comprising an effective amount of a polypeptide that immunologically corresponds to portions of the rhinovirus, interferon-gamma as an adjuvant and a physiologically tolerable carrier and/or diluent, said vaccine when introduced into a host, being capable of inducing the production of antibodies and priming memory cells in the host, said antibodies immunoreacting with said rhinovirus and said vaccine protecting the host from rhino-viral infection.

6. A vaccine as claimed in claim 1 against infection by polio virus comprising an effective amount of a polypeptide that immunologically corresponds to portions of the polio virus, interferon-gamma as an adjuvant and a physiologically tolerable carrier and/or diluent, said vaccine when introduced into a host, being capable of inducing the production of antibodies and priming memory cells in the host, said antibodies immunoreacting with said polio virus and said vaccine protecting the host from polio viral infection.

7. A vaccine as claimed in claim 1 against infection by papilloma virus comprising an effective amount of a polypeptide that immunologically corresponds to portions of the papilloma virus, interferon-gamma as an adjuvant and a physiologically tolerable carrier and/or diluent, said vaccine when introduced into a host, being capable of inducing the production of antibodies and priming memory cells in the host, said antibodies immunoreacting with said papilloma virus and said vaccine protecting the host from papilloma viral infection.

8. A vaccine as claimed in claim 1 against infection by retro viruses comprising an effective amount of a polypeptide that immunologically corresponds to portions of the retro virus, interferon-gamma as an adjuvant and a physiologically tolerable carrier and/or diluent, said vaccine when introduced into a host, being capable of inducing the production of antibodies and priming memory cells in the host, said antibodies immunoreacting with said retro viruses and said vaccine protecting the host from retro-viral infection.

9. A vaccine as claimed in claim 1 against infection by Plasmodium sporozoites comprising an effective amount of an antigenic and immunogenic preparation of the Plasmodium organism, interferon-gamma as an adjuvant and a physiologically tolerable carrier and/or diluent, said vaccine when introduced into a host, being capable of inducing the production of antibodies and priming memory cells in the host, said antibodies immunoreacting with said Plasmodium sporozoites and said vaccine protecting the host from Plasmodium sporozoites infection.

10. A vaccine as claimed in claim 1 against infection by trypanosomes comprising an effective amount of an antigenic and immunogenic preparation of the trypanosome organism, interferon-gamma as an adjuvant and a physiologically tolerable carrier and/or diluent, said vaccine when introduced into a host, being capable of inducing the production of antibodies and priming memory cells in the host, said antibodies immunoreacting with said trypanosomes and said vaccine protecting the host from trypanosome infection.

11. A vaccine as claimed in one of the preceding claims wherein the interferon is human interferon.

12. A vaccine as claimed in one of the preceding claims wherein the interferon is produced by recombinant DNA techniques.

13. A method of preparing a vaccine as claimed in any one of the preceding claims comprising providing an effective amount of an antigenic and immunogenic preparation of the infectious organism and interferon-gamma as an adjuvant and dissolving or dispersing an effective amount of said components in a physiologically tolerable carrier and/or diluent.

14. A method of preparing a vaccine as claimed in claim 13 wherein the effective amount of the antigenic and immunogenic preparation of the infectious organism and the effective amount of interferon-gamma as an adjuvant is separately dissolved or dispersed in an effective amount of a physiologically tolerable carrier and/or diluent and both components are mixed just before injection or are separately administered.

15. The use of interferon-gamma for the preparation of vaccines for protecting hosts from infections by infectious organisms as claimed in one of claims 1 to 10, said vaccines comprising at least two components one of which is an antigenic and immunogenic preparation of the infectious organism and the other is the interferon as an adjuvant.

16. A kit for vaccines as claimed in one of the claims 1 to 10 comprising ampoules or the like with
   a. an effective amount of an antigenic and immunogenic preparation of an infectious organism said organism being a protozoon, virus or bacterium,

b. an effective amount of interferon-gamma to act as an adjuvant and

c. a physiologically tolerable carrier and/or diluent for dissolving or dispersing the components of a.) and b.).

**Patentansprüche**

1. Vakzin zur wirksamen Immunisierung eines Säugers gegen Infektion durch einen infektiösen, im Wirt parasitären Organismus, wobei der Organismus ein Protozoon, ein Virus oder ein Bakterium ist, umfassend eine wirksame Menge einer antigenen und immunogenen Präparation des infektiösen Organismus oder der biologisch aktiven Substanz, Interferon-gamma (IFN-γ) als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit dem infektiösen Organismus oder mit der biologisch aktiven Substanz immunologisch reagieren und wobei das Vakzin den Wirt vor Infektion schützt.

2. Vakzin nach Anspruch 1 gegen Hepatitis B-Virus-Infektion, umfassend eine wirksame Menge eines Polypeptids, welches Teilen des Oberflächenantigens des Hepatitis B-Virus immunologisch entspricht, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit dem Hepatitis B-Virus immunologisch reagieren und das Vakzin den Wirt vor Hepatitis B-Virus-Infektion schützt.

3. Vakzin nach Anspruch 1 gegen Tetanus-Bakterium-Infektion, umfassend eine wirksame Menge einer antigenen und immunogenen Präparation des Tetanus-Bakteriums, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit dem Tetanus-Bakterium immunologisch reagieren und das Vakzin den Wirt vor Tetanus-Bakterien-Infektion schützt.

4. Vakzin nach Anspruch 1 gegen Influenza-Virus-Infektion, umfassend eine wirksame Menge eines Polypeptids, welches Teilen des Influenza-Virus immunologisch entspricht, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit dem Influenza-Virus immunologisch reagieren und das Vakzin den Wirt vor Influenza-Virus-Infektion schützt.

5. Vakzin nach Anspruch 1 gegen Rhinovirus-Infektion, umfassend eine wirksame Menge eines Polypeptids, welches Teilen des Rhinovirus immunologisch entspricht, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit dem Rhinovirus immunologisch reagieren und das Vakzin den Wirt vor Rhinovirus-Infektion schützt.

6. Vakzin nach Anspruch 1 gegen Poliovirus-Infektion, umfassend eine wirksame Menge eines Polypeptids, welches Teilen des Poliovirus immunologisch entspricht, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit dem Poliovirus immunologisch reagieren und das Vakzin den Wirt vor Poliovirus-Infektion schützt.

7. Vakzin nach Anspruch 1 gegen Papillomavirus-Infektion, umfassend eine wirksame Menge eines Polypeptids, welches Teilen des Papillomavirus immunologisch entspricht, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit dem Papillomavirus immunologisch reagieren und das Vakzin den Wirt vor Papillomavirus-Infektion schützt.

**EP 0 241 725 B1**

8. Vakzin nach Anspruch 1 gegen Retroviren-Infektion, umfassend eine wirksame Menge eines Polypeptids, welches Teilen des Retrovirus immunologisch entspricht, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit den Retroviren immunologisch reagieren und das Vakzin den Wirt vor Retrovirus-Infektion schützt.

9. Vakzin nach Anspruch 1 gegen Plasmodium-Sporozoiten-Infektion, umfassend eine wirksame Menge einer antigenen und immunogenen Präparation des Plasmodium-Organismus, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit Plasmodium-Sporozoiten immunologisch reagieren und das Vakzin den Wirt vor Plasmodium-Sporozoiten-Infektion schützt.

10. Vakzin nach Anspruch 1 gegen Trypanosomen-Infektion, umfassend eine wirksame Menge einer antigenen und immunogenen Präparation des Trypanosomen-Organismus, Interferon-gamma als Adjuvans und einen physiologisch verträglichen Träger und/oder ein Verdünnungsmittel, wobei das Vakzin bei Einführung in den Wirt zur Induktion der Produktion von Antikörpern und zur Prägung von Gedächniszellen im Wirt befähigt ist, wobei die Antikörper mit den Trypanosomen immunologisch reagieren und das Vakzin den Wirt vor Trypanosomen-Infektion schützt.

11. Vakzin nach einem der vorhergehenden Ansprüche, worin das Interferon Human-Interferon ist.

12. Vakzin nach einem der vorhergehenden Ansprüche, worin das Interferon durch DNA-Rekombinationstechniken hergestellt wurde.

13. Verfahren zur Herstellung eines Vakzins nach einem der vorhergehenden Ansprüche, wobei man eine wirksame Menge einer antigenen und immunogenen Präparation des infektiösen Organismus und Interferon-gamma als Adjuvans bereitstellt und eine wirksame Menge dieser Komponenten in einem physiologisch verträglichen Träger und/oder einem Verdünnungsmittel löst.

14. Verfahren zur Herstellung eines Vakzins nach Anspruch 13, worin die wirksame Menge der antigenen und immunogenen Präparation des infektiösen Organismus und die wirksame Menge von Interferon-gamma als Adjuvans voneinander getrennt gelöst oder dispergiert sind in einer wirksamen Menge des physiologisch verträglichen Trägers und/oder des Verdünnungsmittels und beide Komponenten vor der Injektion vermischt werden oder getrennt verabreicht werden.

15. Verwendung von Interferon-gamma zur Herstellung von Vakzinen zum Schutz des Wirts vor Infektionen durch infektiöse Organismen nach einem der Ansprüche 1 bis 10, wobei die Vakzine wenigstens zwei Komponenten umfassen, wobei eine davon eine antigene und immunogene Präparation des infektiösen Organismus und die andere Interferon als Adjuvans ist.

16. Kit für Vakzine nach einem der Ansprüche 1 bis 10, umfassend Ampullen oder dergleichen mit
    a) einer wirksamen Menge einer antigenen und immunogenen Präparation eines infektiösen Organismus, wobei der Organismus ein Protozoon, ein Virus oder ein Bakterium ist,
    b) eine wirksame Menge von als Adjuvans wirkendem Interferon-gamma und
    c) einen physiologisch verträglichen Träger und/oder ein Verdünnungsmittel zum Lösen oder Dispergieren der Komponenten gemäß a) und b).

**Revendications**

1. Vaccin pour l'immunisation efficace d'un mammifère contre une infection par un organisme infectieux parasite de l'hôte, ledit organisme étant un protozoaire, un virus ou une bactérie, comprenant une quantité efficace d'une préparation antigénique et immunogénique de l'organisme infectieux ou de la substance biologiquement active, d'interféron gamma (IFN$\gamma$) en tant qu'adjuvant et un véhicule et/ou diluant physiologiquement tolérable, ledit vaccin étant capable, lorsqu'il est introduit dans l'hôte, d'induire chez l'hôte la production d'anticorps et de cellules déclenchant la mémoire, lesdits anticorps immunoréagissant avec ledit organisme infectieux ou avec la substance biologiquement active et ledit

14

vaccin protégeant l'hôte contre une infection.

2. Vaccin selon la revendication 1 contre une infection par le virus de l'hépatite B, comprenant une quantité efficace d'un polypeptide qui correspond du point de vue immunologique à des parties de l'antigène de surface du virus de l'hépatite B, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec ledit virus de l'hépatite B et ledit vaccin protégeant l'hôte contre une infection par le virus de l'hépatite B.

3. Vaccin selon la revendication 1 contre une infection par la bactérie tétanique, comprenant une quantité efficace d'une préparation antigénique et immunogénique de la bactérie tétanique, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec ladite bactérie tétanique et ledit vaccin protégeant l'hôte contre une infection par la bactérie tétanique.

4. Vaccin selon la revendication 1 contre une infection par le virus de la grippe, comprenant une quantité efficace d'un polypeptide qui correspond du point de vue immunologique à des parties du virus de la grippe, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec ledit virus de la grippe et ledit vaccin protégeant l'hôte contre une infection par le virus de la grippe.

5. Vaccin selon la revendication 1 contre une infection par le rhinovirus, comprenant une quantité efficace d'un polypeptide qui correspond du point de vue immunologique à des parties du rhinovirus, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec ledit rhinovirus et ledit vaccin protégeant l'hôte contre une infection par le rhinovirus.

6. Vaccin selon la revendication 1 contre une infection par le poliovirus, comprenant une quantité efficace d'un polypeptide qui correspond du point de vue immunologique à des parties du poliovirus, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec ledit poliovirus et ledit vaccin protégeant l'hôte contre une infection par le poliovirus.

7. Vaccin selon la revendication 1 contre une infection par le virus du papillome, comprenant une quantité efficace d'un polypeptide qui correspond du point de vue immunologique à des parties du virus du papillome, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec ledit virus du papillome et ledit vaccin protégeant l'hôte contre une infection par le virus du papillome.

8. Vaccin selon la revendication 1 contre une infection par les rétrovirus, comprenant une quantité efficace d'un polypeptide qui correspond du point de vue immunologique à des parties du rétrovirus, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec lesdits rétrovirus et ledit vaccin protégeant l'hôte contre une infection par les rétrovirus.

9. Vaccin selon la revendication 1 contre une infection par les sporozoïtes de Plasmodium, comprenant une quantité efficace d'une préparation antigénique et immunogénique de l'organisme Plasmodium, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec lesdits sporozoïtes de Plasmodium et ledit vaccin protégeant l'hôte contre une infection par les sporozoïtes de Plasmodium.

**10.** Vaccin selon la revendication 1 contre une infection par les trypanosomes, comprenant une quantité efficace d'une préparation antigénique et immunogénique de l'organisme trypanosome, d'interféron gamma en tant qu'adjuvant et un véhicule et/ou diluant physiologiquement tolérable, ledit vaccin étant capable, lorsqu'il est introduit dans un hôte, d'induire chez l'hôte la production d'anticorps et de cellules déclenchant la mémoire, lesdits anticorps immunoréagissant avec lesdits trypanosomes et ledit vaccin protégeant l'hôte contre une infection par les trypanosomes.

**11.** Vaccin selon l'une quelconque des revendications précédentes dans lequel l'interféron est un interféron humain.

**12.** Vaccin selon l'une quelconque des revendications précédentes dans lequel l'interféron est produit par des techniques de recombinaison d'ADN.

**13.** Procédé de préparation d'un vaccin selon l'une quelconque des revendications précédentes, comprenant la fourniture d'une quantité efficace d'une préparation antigénique et immunogénique de l'organisme infectieux et d'interféron gamma en tant qu'adjuvant et la dissolution ou la dispersion d'une quantité efficace desdits composants dans un véhicule et/ou diluant physiologiquement tolérable.

**14.** Procédé de préparation d'un vaccin selon la revendication 13 dans lequel la quantité efficace de la préparation antigénique et immunogénique de l'organisme infectieux et la quantité efficace d'interféron gamma en tant qu'adjuvant sont dissoutes ou dispersées séparément dans une quantité efficace d'un véhicule et/ou diluant physiologiquement tolérable et les deux composants sont mélangés juste avant l'injection ou sont administrés séparément.

**15.** Utilisation de l'interféron gamma pour la préparation de vaccins pour la protection d'hôtes contre des infections par des organismes infectieux selon l'une quelconque des revendications 1 à 10, lesdits vaccins comprenant au moins deux composants dont l'un est une préparation antigénique et immunogénique de l'organisme infectieux et l'autre est l'interféron en tant qu'adjuvant.

**16.** Trousse pour des vaccins selon l'une des revendications 1 à 10, comprenant des ampoules ou analogues contenant
a. une quantité efficace d'une préparation antigénique et immunogénique d'un organisme infectieux, ledit organisme étant un protozoaire, un virus ou une bactérie,
b. une quantité efficace d'interféron gamma destiné à jouer le rôle d'adjuvant et
c. un véhicule et/ou diluant physiologiquement tolérable pour dissoudre ou disperser les composants de a.) et b.).

Fig. 1

Fig. 2

Fig. 3